# EUROPEAN PATENT APPLICATION

(11) **EP 4 506 462 A1**
(43) Date of publication of application: **12.02.2025**
(21) Application number: 23778402.0
(22) Date of filing: 30.03.2023
(51) Int. Cl.: C12P 13/04, C12N 15/60, C12N 15/70, C12N 1/21, C12P 19/32, C12N 15/53

(54) **METHOD FOR PREPARING GLYCINE, ACETYL COENZYME A, AND ACETYL COENZYME A DERIVATIVE BY USING THREONINE**

(30) Priority: 02.04.2022 CN 202210352016
(71) Applicant: Mint Biotechnologies Co., Ltd., Hangzhou, Zhejiang 310012 (CN)
(72) Inventor: NIE, Mengzhen, Hangzhou, Zhejiang 310012 (CN); ZHANG, Kechun, Hangzhou, Zhejiang 310012 (CN)
(74) Representative: Gille Hrabal Partnerschaftsgesellschaft mbB Patentanwälte
(86) International application number: PCT/CN2023/085219
(87) International publication number: WO 2023/186037

(57) **Abstract**

Provided is a method for preparing glycine by using threonine. In a fermentation process, threonine is decomposed into glycine and acetaldehyde by aldolase. Also provided is a method for simultaneously preparing glycine and acetyl coenzyme A. In a fermentation process, acetaldehyde is reduced into acetyl coenzyme A or an acetyl coenzyme A derivative by acetylating acetaldehyde dehydrogenase; or threonine is dehydrogenated by threonine dehydrogenase to obtain 2-amino-3-ketobutyric acid, which is then ligated by 2-amino-3-ketobutyrate CoA ligase to obtain acetyl coenzyme A, and acetyl coenzyme A can be converted into an acetyl coenzyme A derivative under different fermentation conditions. The present disclosure features a unique concept, enabling the preparation of glycine, acetyl-CoA, and acetyl-CoA derivatives with high conversion efficiency, shorter time consumption, high yield, abundant products, and suitability for industrial processing and production.

## Description

### CROSS-REFERENCE TO RELATED APPLICATIONS

**The present disclosure claims priority to the prior application with the patent application No.** 202210352016.3 entitled "METHOD FOR PREPARING GLYCINE, ACETYL COENZYME A, AND ACETYL COENZYME A DERIVATIVE BY USING THREONINE" and filed with the China National Intellectual Property Administration on April 2, 2022**, which is incorporated herein by reference in its entirety.**

### TECHNICAL FIELD

The present disclosure relates to the technical field of genetic engineering and fermentation engineering, in particular to a method for preparing glycine, acetyl-CoA, and an acetyl-CoA derivative by using threonine.

### BACKGROUND

Acetyl coenzyme A (acetyl-CoA), which is composed of coenzyme A (CoA) and acetate ligated by a thioester bond, is one of the important intermediate metabolites in cellular metabolism and participates in a variety of cellular life processes. Acetyl-CoA is produced by the catabolism of sugars, fats, amino acids, etc., and finally is completely oxidized through the tricarboxylic acid cycle and oxidative phosphorylation to generate carbon dioxide and water, releasing energy for the synthesis of ATP. In addition, it can be used as a precursor of many important biological macromolecules, including fatty acid, 1-butanol, polyhydroxyalkanoic acid, polyketonic acid, isoprene, etc. Fatty acid can be used for dietary supplements, drugs, biodiesel, etc.; polyketonic acid can be used in pharmaceuticals; and isoprene can be used for manufacturing essences, spices, biofuels, dietary supplements, vitamins, drugs, etc. However, efficient biosynthesis of these important biological macromolecules is often hampered by the insufficient supply of acetyl-CoA. Therefore, it is of paramount importance how the yield of acetyl-CoA is increased to ensure the supply.

Under aerobic conditions, the decarboxylation of pyruvic acid produced from glycolysis is one of the major pathways for the synthesis of acetyl-CoA. In addition, exogenous fatty acid continuously shortens the carbon chain through β-oxidation to finally form acetyl-CoA; or certain amino acid metabolism is also one of the pathways providing acetyl-CoA. In microorganisms such as *Escherichia coli,* one of the most direct methods for increasing the yield of acetyl-CoA is through overexpression of acetyl-CoA synthetase and knockout of competitive pathways. By utilizing the overexpression of various genes and the optimization of metabolic pathways, various derivatives of Acetyl-coA, such as terpenes, 1-butanol, polyhydroxyalkanic acid, and the like, can be synthesized. Kang Zhou et al. converted ethanol into acetyl-CoA in *Escherichia coli* using acetylating acetaldehyde dehydrogenase (ada) of *Dickeya zeae* and alcohol dehydrogenase (adh2) of *Saccharomyces cerevisiae* without consuming ATP, and used it as a platform for the synthesis of PHB in a yield of 1.1 g/L. Javier Cardenas et al. enhanced the synthesis of acetyl-CoA and NADPH/NADP+ by blocking the pentose phosphate pathway and modifying pyruvate dehydrogenase in *Escherichia coli,* thereby increasing the yield of polyketide to 1.6 g/L. Menghao Cai et al., using a short and strong ethanol assimilation pathway, directly produced acetyl-CoA in Crabtree-negative yeasts. This method can be used for synthesizing the acetyl-CoA derivative drug monacolin with a yield of 2 g/L. However, currently, methods for increasing the yield of acetyl-CoA within bacteria through microbial metabolic engineering are relatively not diverse and generally characterized by low conversion efficiency, long time consumption and low yield. These methods cannot truly overcome the problem of limited synthesis yield of many important macromolecules due to insufficient supply of acetyl-CoA at an industrial scale, and they are in urgent need of improvement.

Glycine is an important starting material in industries of chemical engineering, pharmaceuticals, pesticides, etc., with high yield and wide application. Currently, in the methods for producing glycine, mostly α-chloroacetic acid or the Strecker method are used. There are patent documents which describe that glycine can be isolated with high content in the presence of urotropine and organic amine by using chloroacetic acid and ammonia as starting materials, but a large amount of sodium alkoxide or potassium alkoxide is required for recycling effective components in the mother liquor. Thus, this method has low economic value; meanwhile, recycling excessive alcohol leads to increased energy consumption and higher investment, as well as the generation of salt mud, which is relatively troublesome for subsequent treatment. Therefore, the present disclosure aims to develop a method for co-producing glycine and acetyl-CoA or an acetyl-CoA derivative to better meet the actual need.

### SUMMARY

The technical problem to be solved by the present disclosure is to provide a method for preparing glycine, acetyl-CoA, and an acetyl-CoA derivative by using threonine so as to improve the yield and transformation rate of glycine and acetyl-CoA or an acetyl-CoA derivative, and the method provided is more suitable for industrial production.

The technical problem solved by the present disclosure is realized by adopting the following technical solutions:
In a first aspect, the present disclosure provides a recombinant microorganism for fermentatively producing glycine by using threonine, and the microorganism overexpresses or exogenously expresses an aldolase gene.

In a specific embodiment of the present disclosure, the aldolase gene comprises a threonine aldolase gene or a phenylserine aldolase gene, and the threonine aldolase gene or the phenylserine aldolase gene comprises one or more of ltaE, TdcB, glyA, Sdsl, bhcC, psald, pdxA, or vrtJ.

In a second aspect, the present disclosure provides a recombinant microorganism for fermentatively producing glycine and acetyl-CoA or a derivative thereof by using threonine, wherein the recombinant microorganism at least comprises an aldolase gene and an acetylating acetaldehyde dehydrogenase gene, and/or the recombinant microorganism at least comprises a threonine dehydrogenase gene and a 2-amino-3-ketobutyrate CoA ligase gene;
optionally, the expression activity of an alcohol dehydrogenase of the microorganism is weakened or eliminated, and encoding genes for the alcohol dehydrogenase are one or more of yqhD, qbdA, adhE, or mdH;
optionally, the expression activity of a pyruvate dehydrogenase or a lactate dehydrogenase of the microorganism is weakened or eliminated, and the pyruvate dehydrogenase or the lactate dehydrogenase is aceE or ldhA;
preferably, the aldolase gene comprises one or more of ltaE, TdcB, glyA, Sdsl, bhcC, psald, pdxA, or vrtJ; the acetylating acetaldehyde dehydrogenase gene comprises one or more of eutE, bphJ, TTHB247, mhpF, ADH2, or hsaG; the threonine dehydrogenase gene comprises one or more of tdh, ydfG, pdxA, asd, AKHSDH2, or trmG; and the 2-amino-3-ketobutyrate CoAligase gene comprises one or more of kbI, GCAT, Gcat, or TTHA1582;
preferably, the aldolase gene, the acetylating acetaldehyde dehydrogenase gene, the threonine dehydrogenase gene, or the 2-amino-3-ketobutyrate CoA ligase is introduced into a genomic gene;
preferably, the aldolase gene, the acetylating acetaldehyde dehydrogenase gene, the threonine dehydrogenase gene, or the 2-amino-3-ketobutyrate CoA ligase is initiated by a strong promoter;
preferably, the aldolase gene, the acetylating acetaldehyde dehydrogenase gene, the threonine dehydrogenase gene, or the 2-amino-3-ketobutyrate CoA ligase is integrated to positions of aceE, ldhA, or/and adhE;
preferably, Tdh, tdcB, and Yiay genes are deleted in the microorganism;
preferably, gcvTHP gene is deleted in the microorganism.

In a specific embodiment of the present disclosure, the microorganism is used for synthesizing mevalonic acid and expresses three genes of AtoB, MvaS and MvaE by plasmid expression or genome integration;
or
the microorganism is used for synthesizing N-acetyl-glucosamine (Glcnac); nagAB, nagK, and murQ are deleted in the microorganism and the microorganism comprises the following genes: one or any combination of glutamine fructose-6 phosphate transaminase glmS, phosphatase gene yqaB, glutamine synthetase gene glnA, or acetylglucosamine synthetase GNA1.

In a specific embodiment of the present disclosure, the recombinant microorganism is constructed by a genetic engineering method including plasmid expression or genomic integration.

In a specific embodiment of the present disclosure, the recombinant microorganism is constructed by the plasmid expression method, and the construction method is as follows: the gene is amplified by PCR, the obtained gene is ligated to a plasmid vector, and the plasmid vector was then transformed into competent cells; and after sequencing, a recombinant expression plasmid vector is obtained and then transformed into a microorganism to obtain the recombinant microorganism.

In a specific embodiment of the present disclosure, the plasmid is one or two of pZAlac and pZElac, and the competent cells are *E.coli* dh5a competent cells.

In a specific embodiment of the present disclosure, the recombinant expression plasmid vector is pZE-ltaE, and the construction method for the plasmid pZE-ltaE is as follows: an ltaE gene is obtained by PCR amplification using a genome of *Pseudomonas putida* as a template; the ltaE gene is ligated to a pZElac vector comprising an IPTG inducible promoter, and the vector is transformed into *E.coli* dh5a competent cells; and the plasmid pZE-ltaE is obtained after sequencing.

In a specific embodiment of the present disclosure, the recombinant expression plasmid vector is pZE-ltaE_eutE, and the construction method for the pZE-ltaE_eutE is as follows: an eutE gene is obtained by PCR amplification using a genome of *Escherichia coli* MG1655 as a template, and an ltaE gene is obtained by PCR amplification using a genome of *Pseudomonas putida* as a template; the eutE and ltaE genes are ligated to a pZElac vector comprising an IPTG inducible promoter, and the vector is transformed into *E.coli* dh5a competent cells; and the plasmid pZE-ltaE_eutE is obtained after sequencing;
and/or
the recombinant expression plasmid vector is pZE-tdH kbI, and the construction method for the pZE-tdH kbI is as follows: tdH and _kbI genes are obtained by PCR amplification using a genome of *Escherichia coli* MG1655 as a template; the tdH and _kbI genes are ligated to a pZElac vector comprising an IPTG inducible promoter, and the vector is transformed into *E.coli* dh5a competent cells; and the plasmid pZE-tdH kbI is obtained after sequencing.

In a specific embodiment of the present disclosure, the microorganism is selected from one or more of *Escherichia coli, Bacillus, Corynebacterium, yeast,* or *Streptomyces.*

In a specific embodiment of the present disclosure, the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

In a third aspect, the present disclosure provides use of the recombinant microorganism for fermentatively producing glycine by using threonine.

In a fourth aspect, the present disclosure provides a method for preparing glycine by using threonine, wherein threonine is used as a substrate and the recombinant microorganism described above is added for fermentation; and during the fermentation, the threonine is decomposed into glycine and acetaldehyde.

In a specific embodiment of the present disclosure, during the fermentation, a strain of the recombinant microorganism is inoculated into a 2-xyT culture medium containing ampicillin and chloramphenicol for culturing for 3-6 h, and then IPTG is added to a final concentration of 0.2-0.4 mM; the mixture was cultured for another 15-30 h and then centrifuged to obtain a bacterial solution; the bacterial solution was added to a Tris-HCl buffer, and threonine is added at an amount of 15-30 g/L as a substrate; the resulting mixture is fermented to obtain a fermentation broth containing glycine and acetaldehyde.

In a fifth aspect, the present disclosure provides use of the recombinant microorganism described above for fermentatively producing glycine and acetyl-CoA or a derivative thereof by using threonine.

In a sixth aspect, the present disclosure provides a method for preparing glycine and acetyl-CoA by using threonine, wherein threonine is used as a substrate and the recombinant microorganism described above is added for fermentation, during the fermentation, the threonine is decomposed into glycine and acetaldehyde, and the acetaldehyde is directly converted into acetyl-CoA or the acetaldehyde is converted into acetic acid first and then into acetyl-CoA; or the threonine is dehydrogenated to obtain 2-amino-3-ketobutyric acid, and the 2-amino-3-ketobutyric acid is subjected to the action of a 2-amino-3-ketobutyrate CoA ligase to obtain acetyl-CoA.

In a specific embodiment of the present disclosure, during the fermentation, the recombinant microorganism is inoculated into a 2-xyT culture medium containing ampicillin and chloramphenicol but no antibiotics, and threonine is added at an amount of 15-25 g/L as a substrate; the mixture is cultured at 30-35 °C for 3-6 h, and then IPTG is added to a final concentration of 0.2-0.4 mM; the resulting mixture was cultured for another 10-12 h and then centrifuged after the culture is finished, and a supernatant is retained, thus obtaining glycine and acetyl-CoA.

In a seventh aspect, the present disclosure provides a method for preparing glycine and an acetyl-CoA derivative by using threonine, wherein glycine and acetyl-CoA are prepared by the method described above, and the acetyl-CoA is converted into an acetyl-CoA derivative.

In a specific embodiment of the present disclosure, the acetyl-CoA derivative comprises one or more of mevalonic acid, sialic acid, N-acetylglucosamine, 3-hydroxybutyric acid, or fatty acid; preferably, the derivative is mevalonic acid or N-acetylglucosamine.

In a specific embodiment of the present disclosure, the acetyl-CoA derivative is mevalonic acid; during the fermentation, the recombinant microorganism is inoculated into a shake flask containing an M9 culture medium containing ampicillin but no glucose, and threonine is added at an amount of 15-25 g/L as a substrate; the mixture was cultured at 30-35 °C and a rotation speed of 200-300 rpm for 3-5 h, and then IPTG is added to a final concentration of 0.2-0.4 mM; the resulting mixture was cultured and fermented for another 20-30 h, and a fermentation broth is collected.

In a specific embodiment of the present disclosure, the acetyl-CoA derivative is mevalonic acid; during the fermentation, the recombinant microorganism is subjected to an expansion culture and then inoculated into a fermentor containing an M9 culture medium containing ampicillin but no glucose; after 6-8 h of fermentation and culture, IPTG is added to a final concentration of 0. 1-0.2 mM, and threonine is added at an amount of 100-150 g/L as a substrate; the resulting mixture was fermented and cultured at 30-35 °C for another 15-20 h, and a fermentation broth is collected.

In an eighth aspect, the present disclosure provides a recombinant DNA or biomaterial for fermentatively producing glycine and acetyl-CoA or a derivative thereof, wherein the recombinant DNA at least comprises an aldolase gene and an acetylating acetaldehyde dehydrogenase gene, and/or the recombinant microorganism at least comprises a threonine dehydrogenase gene and a 2-amino-3-ketobutyrate CoA ligase gene;
preferably, the aldolase gene comprises one or more of ltaE, TdcB, glyA, Sdsl, bhcC, psald, pdxA, or vrtJ; the acetylating acetaldehyde dehydrogenase gene comprises one or more of eutE, bphJ, TTHB247, mhpF, ADH2, or hsaG; the threonine dehydrogenase gene comprises one or more of tdh, ydfG, pdxA, asd, AKHSDH2, or trmG; and the 2-amino-3-ketobutyrate CoAligase gene comprises one or more of kbI, GCAT, Gcat, or TTHA1582;
the biomaterial is an expression cassette, a transposon, a plasmid vector, a phage vector, or a virus vector.

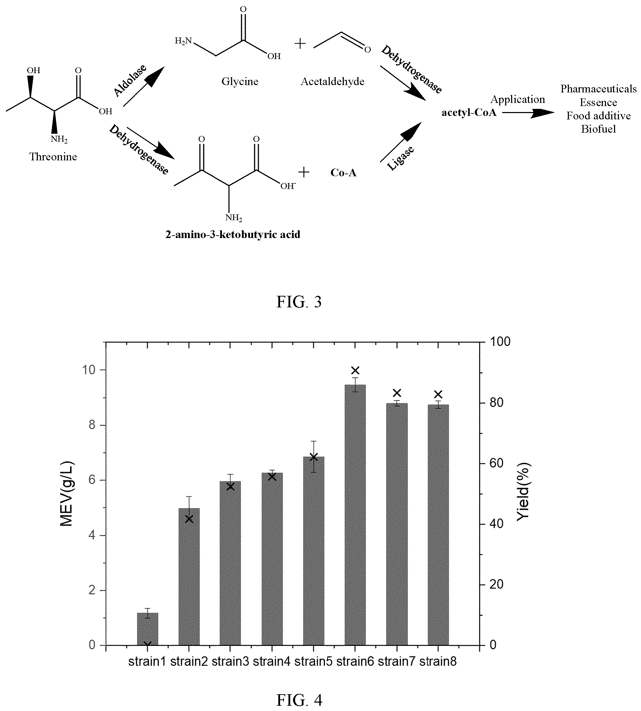

The reaction pathway of the present disclosure is shown in FIG. 3.

Beneficial effects: The method for preparing glycine and acetyl-CoA by using threonine described herein enables obtaining of glycine alone, or obtaining of glycine and acetyl-CoA simultaneously, or obtaining of glycine and an acetyl-CoA derivative simultaneously. This method features high transformation efficiency, less time consumption, high yield, abundant products, and high economic value, and it is easy to realize industrial processing and production.

According to the method for preparing glycine by using threonine described herein, the concentration of glycine generated in the fermentation broth is up to 11.2 g/L. The yield is high, the by-products is few, the economic value is high, and the subsequent treatment cost is low. According to the method for preparing glycine and acetyl-CoA by using threonine described herein, the concentration of acetyl-CoA in the obtained fermentation broth is up to 12-14 ng/L, which is far higher than the yield obtained by directly fermenting wild *Escherichia coli.* According to the method for preparing glycine and an acetyl-CoA derivative by using threonine described herein, in the fermentation broth obtained by fermentation in a shake flask, the concentration of glycine is up to 11 g/L, and the concentration of mevalonic acid serving as one of acetyl-CoA derivatives is up to 3.8 g/L; in the fermentation broth obtained by fermentation in a fermentor, the concentration of glycine is higher than 60 g/L, and the concentration of mevalonic acid serving as one of acetyl-CoA derivatives is higher than 30 g/L.

### BRIEF DESCRIPTION OF THE DRAWINGS

FIG. 1 is a schematic diagram showing the detection results of acetyl-CoA in Example 2;
FIG. 2 is a schematic diagram showing the detection results of the concentrations of glycine and mevalonic acid in fermentation broth at different stages in Example 5
FIG. 3 is a diagram of the reaction pathway of the present disclosure;
FIG. 4 is a comparison of mevalonic acid expression levels in different strains;

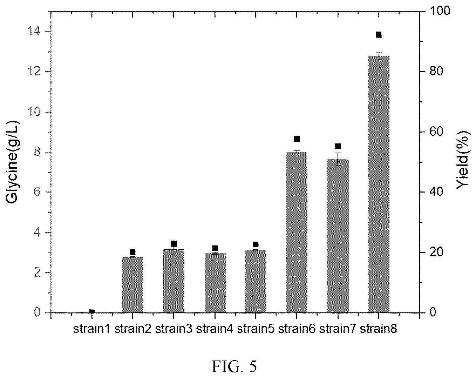

FIG. 5 is a comparison of glycine expression levels in different strains.

### DETAILED DESCRIPTION

In order to make the technical means, creation characteristics, achieved purposes, and effects of the present disclosure easy to understand, the present disclosure is further illustrated below with reference to specific examples.

In the present disclosure, unless otherwise indicated, the scientific and technical terms used herein have the same meaning as commonly understood by those skilled in the art. In addition, the terms and laboratory procedures related to nucleic acid chemistry, molecular biology, cell and tissue culture, microbiology, and immunology used herein are the terms and conventional procedures widely used in the corresponding fields. Also, in order to better understand the present disclosure, the definitions and interpretations of the related terms are provided below. It should be appreciated that the terms used herein are for the purpose of illustrating particular embodiments only, and are not intended to be limiting.

The articles "a", "an" and "the" are used herein to refer to one or more than one of the grammatical object of the article.

The use of alternatives (e.g., "or") should be understood to refer to one, two, or any combination of the alternatives. The term "and/or" should be interpreted as referring to one or both of the alternatives.

As used herein, the term "gene synthesis" refers to a generation process using recombinant DNA techniques or a production process using DNA or amino acid sequence synthesis techniques available and well known in the art.

The term "encode" or "code" refers to the inherent property of specific sequences of nucleotides in a polynucleotide, such as a gene, a cDNA, or an mRNA, to serve as a template in synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of an mRNA corresponding to the gene produces the protein in a cell or other biological system. Both the coding strand, which comprises a nucleotide sequence equivalent to the mRNA sequence and is usually provided in a sequence listing, and the non-coding strand, which is used as a template for transcription of a gene or cDNA, may be referred to as encoding the protein or other product of that gene or cDNA.

As used herein, the term "endogenous" refers to any substance derived from or produced within an organism, cell, tissue or system.

As used herein, the term "exogenous" refers to any substance introduced into or produced outside of an organism, cell, tissue or system.

As used herein, the term "expression" is defined as the transcription and/or translation of a particular nucleotide sequence driven by its promoter.

Unless otherwise specified, the "polynucleotide sequence encoding an amino acid sequence" includes all nucleotide sequences that are degenerate versions of each other and encode the same amino acid sequence. The phrase "nucleotide sequence encoding a protein or an RNA" may also include introns to the extent that the nucleotide sequence encoding the protein may contain an intron(s) in some versions.

As used herein, the term "vector" refers to a composition of matter that comprises an isolated nucleic acid and can be used to deliver the isolated nucleic acid to the interior of a cell. The transferred nucleic acid is typically ligated to, e.g., inserted into, a vector nucleic acid molecule. A vector may comprise sequences that direct autonomous replication in the cell, or may comprise sequences sufficient to allow integration into the host cell DNA. Many vectors are known in the art, including but not limited to plasmids, phagemids, artificial chromosomes, bacteriophages and animal viruses. Therefore, the term "vector" encompasses an autonomously replicating plasmid or virus.

The recipient strain may be *Escherichia coli, Corynebacterium glutamicum, Bacillus subtilis, Bacillus megaterium, Bacillus amyloliquefaciens, Vibrio natriegens, Saccharomyces cerevisiae,* or the like.

### The plasmids of the present disclosure are constructed and operated as follows:

The recombinant expression plasmid vector used in examples is pZE-ltaE, and the construction method is as follows:
An ltaE gene was obtained by PCR amplification using a genome of *Pseudomonas putida* as a template:
ltaE-F(GAATTCATTAAAGAGGAGAAAGGTACCATGACAGACAAGAGCCAACAATTCGCC AGCG , SEQ ID NO:1)/ltaE-R(CTTTCGTTTTATTTGATGCCTCTAGATCAGCCACCAATGATCGTGCGGATA , SEQ ID NO:2)

The ltaE gene was ligated to a pZElac vector comprising an IPTG inducible promoter by a non-ligase-dependent single-fragment quick cloning kit. The pZElac vector was firstly heat-transformed into *E.coli* dh5a competent cells by a standard method, and an ampicillin resistance plate was coated with the cells for culturing overnight. Positive clones were selected for sequencing verification, and the recombinant plasmid vector verified to be correct by sequencing was named plasmid pZE-ltaE.

The recombinant expression plasmid vector used in examples is pZE-ltaE_eutE, and the construction method is as follows:
A primer was designed based on the genomic sequence of *Pseudomonas putida* published by NCBI:
ltaE-F'(GAATTCATTAAAGAGGAGAAAGGTACCATGACAGACAAGAGCCAACAATTCGC CAGCG , SEQ ID NO:3)/ltaE-R'(GATTCATACTAGTAGTTAATTTCTCCTTCAGCCACCAATGATCGTGCGGATATCCG C, SEQ ID NO:4);
A primer was designed based on the genomic sequence of *Escherichia coli* MG1655 published by NCBI:
   eutE-F(ATTGGTGGCTGAAGGAGAAATTAACTACTAGTATGAATCAACAGGATATTGAAC, SEQ ID NO:5)/eutE-R(TTTCGTTTTATTTGATGCCTCTAGATTAAACAATGCGAAACGCATCGACTAATAC, SEQ ID NO:6);
An ltaE gene was obtained by PCR amplification using the genome of *Pseudomonas putida* as a template, and the eutE gene was obtained by PCR amplification using the genome of *Escherichia coli* MG1655 as a template. The ltaE and eutE genes were ligated to a pZElac vector comprising an IPTG inducible promoter by a non-ligase-dependent single-fragment quick cloning kit. The pZElac vector was firstly heat-transformed into *E.coli* dh5a competent cells by a standard method, and an ampicillin resistance plate was coated with the cells for culturing overnight. Positive clones were selected for sequencing verification, and the recombinant plasmid vector verified to be correct by sequencing was named pZE-ltaE_eutE. The *E.coli* dh5a competent cells can fully express plasmids after undergoing gene editing, and are suitable for plasmid sequencing and plasmid amplification.

The recombinant expression plasmid vector used in examples is pZE-tdH_kbI, and the construction method is as follows:
A primer was designed based on the genomic sequence of *Escherichia coli* MG1655 published by NCBI:
tdH-F(GAATTCATTAAAGAGGAGAAAGGTACCATGAAAGCGTTATCCAAACTGAAAG , SEQ ID NO:7)/tdH-R(TCTCCACGCATAGTTAATTTCTCCTTTAATCCCAGCTCAGAATAACTTTC, SEQ ID NO:8);
kbI-F(GAAAGTTATTCTGAGCTGGGATTAAAGGAGAAATTAACTATGCGTGGAGA , SEQ ID NO:9)/kbI-R(TTTCGTTTTATTTGATGCCTCTAGATCAGGCGATAACGCCCAGTTGTTTA, SEQ ID NO:10);
Genes tdH and kbI were obtained by PCR amplification using the genome of *Escherichia coli* MG1655 as a template and were ligated to a pZElac vector comprising an IPTG inducible promoter by a non-ligase-dependent single-fragment quick cloning kit. The pZElac vector was firstly heat-transformed into *E.coli* dh5a competent cells by a standard method, and an ampicillin resistance plate was coated with the cells for culturing overnight. Positive clones were selected for sequencing verification, and the recombinant plasmid vector verified to be correct by sequencing was named pZE-tdH _kbI.

The recombinant expression plasmid vector used in examples is Pze-ggGy, and the construction method is as follows:
A primer was designed based on the genomic sequence of *Escherichia coli* MG1655 published by NCBI:
glmS-F(aattcattaaagaggagaaaggtaccATGTGTGGAATTGTTGGCGCGATCG , SEQ ID NO:11)/ glmS-R(GACATAGTTAATTTCTCCTaagcttTTACTCAACCGTAACCGATTTTGCC , SEQ ID NO:12);
yqaB-F(GCTGTACTACAGCGTCTAAactagtAGGAGAAATTAACTATGTACGAGCGTTATGCA GGTTTAAT , SEQ ID NO: 13)/yqaB-R(CTCATAGTTAATTTCTCCTagatctTCACAGCAAGCGAACATCCACGGCG , SEQ ID NO:14);
glnA-F(ATCGGTTACGGTTGAGTAAaagcttAGGAGAAATTAACTATGTCCGCTGAACACGTA CTGACGAT , SEQ ID NO:15)/glnA-R(TACATAGTTAATTTCTCCTactagtTTAGACGCTGTAGTACAGCTCAAAC , SEQ ID NO:16;
A primer is designed based on the genomic sequence of *Saccharomyces cerevisiae* published by NCBI:
   GNA1-F(GGATGTTCGCTTGCTGTGAagatctAGGAGAAATTAACTATGAGCTTACCCGATGGA TTTTATAT , SEQ ID NO:17)/GNA1-R(cgttttatttgatgcctctagagctagcCTATTTTCTAATTTGCATTTCCACG, SEQ ID NO: 18) Glutamine-fructose-6-phosphate transaminase (glmS), yqaB, and glnA genes were obtained by PCR amplification using the genome of *Escherichia coli* MG1655 as a template, and the GNA1 gene was obtained by PCR amplification using the genome of *Saccharomyces cerevisiae* as a template. These genes were ligated to a pZElac vector comprising an IPTG inducible promoter by a non-ligase-dependent single-fragment quick cloning kit. The pZElac vector was firstly heat-transformed into *E.coli* dh5a competent cells by a standard method, and an ampicillin resistance plate was coated with the cells for culturing overnight. Positive clones were selected for sequencing verification, and the recombinant plasmid vector verified to be correct by sequencing was named pZE-ggGy. The *E.coli* dh5a competent cells can fully express plasmids after undergoing gene editing, and are suitable for plasmid sequencing and plasmid amplification.

### The genome operation of the present disclosure is as follows:

### Gene knockout

Taking the deletion of gene aceE from a wild-type strain(strain 1) as an example, the construction method is as follows:
A primer was designed based on the genomic sequence of *Escherichia coli* MG1655 published by NCBI:
AceE-F (ATGTCAGAACGTTTCCCAAATGACGTGGTGTAGGCTGGAGCTGCTTC, SEQ ID NO:19)/AceE-R (ACGCCAGACGCGGGTTAACTTTATCTGCATCATTCCGGGGATCCGTCGACC, SEQ ID NO:20). pKD13 (pkd13 is a knockout system vector containing a kan marker) was amplified by using aceE-F and aceE-R primers, with homologous regions of the gene aceE on both sides. Then, the DNA fragment was electroporated into wild type *Escherichia coli* BW25113 by using pKD46 to obtain a kan marker-containing strain with a gene deletion, and the colonies containing the deletion of acee were transformed by using plasmid pCP20 to remove the kanamycin resistance marker.

### Gene integration:

A method for integrating a acetyl-CoA synthesis pathway (an ltaE_eutE operon regulated by a strong promoter) into a genome by using red homologous recombination (taking the integration and replacement of aceE gene to obtain strain2 as an example):
A primer was designed based on the genomic sequence of *Escherichia coli* MG1655 published by NCBI:

aceE-F1(GCTTTCCGGCGAGAGTTCAATGGGTGTAGGCTGGAGCTGCTTCGAAGT，
SEQ ID NO:21)/ aceE-
R1(GTTAATTTCTCCTGTTTAAACGTACATGCTAACAATACGGGCTCAATTATATCA
ACGTTG，SEQ ID NO:22)
aceE-
F2(GTTAGCATGTACGTTTAAACAGGAGAAATTAACTATGACAGACAAGAGCCAA
CAATTCG，SEQ ID NO:23)/aceE-
R2(CTTTATCTGCATCGATGTTGAATTTGGCTTAAACAATGCGAAACGCATCGACT
，SEQ ID NO:24)

A pKD13 fragment was amplified by using aceE-F1 and aceE-R1 primers, and the fragment contained a strong promoter M1-93 (TTATCTCTGGCGGTGTTGACAAGAGATAACAACGTTGATATAATTGAGCCCGTATT GTTAGCATGTACGTTTAAACAGGAGAAATTAACT , SEQ ID NO:25); an ltaE_eutE fragment was amplified by using aceE-F2 and aceE-R2 primers. Using the two fragments above as templates, an ltaE_eutE operon with a kan marker was obtained by PCR with aceE-F 1/aceE-R2 as primers. This fragment had homologous regions of aceE on both sides. Then, the DNA fragment was electroporated into strain1 by using pKD46 to obtain a kan marker-containing strain with a gene deletion. The colonies containing the deletion of ltaE_eutE were transformed by plasmid pCP20 to remove the kanamycin resistance marker, thus obtaining strain2.

The acetyl-CoA synthesis pathway (ltaE_eutE operon) was integrated into a chromosome by using a similar method to replace lactate dehydrogenase gene (ldhA) or aldehyde-alcohol dehydrogenase (adhE).

The biomaterials constructed by the present disclosure are as follows:

| **Name** | **Introduced gene** | **Recipient material or microorganism** |
|---|---|---|
| pZE-ltaE | ltaE | pZElac |
| pZE-ltaE_eutE | ltaE eutE | pZElac |
| pZE-tdH_kbI | tdH, kbI | pZElac |
| pZE-ggGy | glmS, yqaB, glnA, GNA1 | pZElac |
| strain 1 | Deletion of aceE | Wild type *Escherichia coli* |
| strain 2 | Integration of ltaE_eutE to aceE location | Wild type *Escherichia coli* |
| strain 3 | Deletion of tdh | Strain2 |
| strain 4 | Deletion of tdcB | Strain3 |
| strain 5 | Deletion of yiay | Strain4 |
| strain 6 | Integration of ItaE_eutE to ldhA position | strain 5 |
| strain 7 | Integration of ItaE_eutE to adhE location | strain 6 |
| strain 8 | Deletion of gcvTHP | Strain7 |
| strain 9 | Deletion of nagAB, nagK, and murQ | strain 2 |
| strain 10 | Integration of ItaE_eutE to ldhA position | strain 9 |
| strain 11 | Integration of ItaE_eutE to adhE location | strain 10 |

In the present disclosure, the *Escherichia coli* CMEV-1 is selected from the strains disclosed in the document previously published by the applicant, and the document number is DOI: 10.1128/AEM.02178-16. After the gene knockout in *Escherichia coli,* the expression activity of alcohol dehydrogenase is weakened or eliminated, which can reduce the consumption of acetyl-CoA, thereby increasing the yield of products.

In the present disclosure, the plasmid pMEV-1 is selected from plasmids disclosed in the document previously published by the applicant, and the document number is DOI: 10.1073/pnas.1404596. The function of the plasmid is to obtain mevalonic acid by converting acetyl-coA through three enzymes of AtoB, MvaS, and MvaE in sequence.

### Example 1

The recombinant expression plasmid vector pZE-ltaE was transformed into an expression strain of *Escherichia coli* BL21, and the strain was inoculated into a 2-xyT culture medium containing ampicillin and chloramphenicol. The mixture was cultured in a 50 mL Erlenmeyer flask (medium volume 10 mL) at 30 °C and a rotation speed of 240 rpm for 3-6 h. IPTG was added to a final concentration of 0.3 mM, and the mixture was cultured for another 20 h to induce protein expression. The bacteria were centrifuged at 4 °C with a rotation speed of 8000 rpm for 5 min. The culture supernatant was discarded, and the bacterial solution was subjected to an ice bath for further use.

The bacterial solution described above was added to a 2 mL system containing 50 mM Tris-HCl buffer (pH 7.5) and 20 g/L threonine, and the mixture was allowed to react under shaking in a shaker at 30 °C and 240 rpm for 20 h to obtain a transformation solution containing glycine and acetaldehyde. After the reaction was finished, the obtained transformation solution was diluted 20-fold by using deionized water and centrifuged at 12500 rpm for 10 -15 min. The supernatant was pipetted and diluted, and high performance liquid chromatography analysis was performed. The concentrations of the substrate and products in the catalytic system were determined. It was determined that after 20 h of catalytic reaction, the system generated glycine at a concentration of 11.8 g/L, with a yield reaching 93.6%, and acetaldehyde at a concentration of 2.7 g/L. In the process, the acetaldehyde was partially oxidized and reduced. The yield was calculated by the formula: (actual yield/theoretical yield) × 100%.

### Example 2

The recombinant expression plasmid vector pZE-ltaE_eutE was transformed into wild type *Escherichia coli,* and the strain was inoculated into a 2-xyT culture medium containing ampicillin and chloramphenicol. Threonine was added at an amount of 20 g/L as a substrate, and the mixture was cultured in a 50 mL Erlenmeyer flask (medium volume 10 mL) at 30 °C and a rotation speed of 240 rpm for 3-6 h. IPTG was added to a final concentration of 0.3 mM, and the mixture was cultured for another 10 h for protein expression. The bacteria were subjected to cell wall breaking at 4 °C and centrifuged at a rotation speed of 8000 rpm for 5 min. The culture supernatant was retained to obtain glycine and acetyl-CoA. It was detected that the generated glycine concentration was 10.8 g/L, with a yield reaching 85.7%, and the acetyl-CoA concentration was 18.53 ng/L.

### Example 3

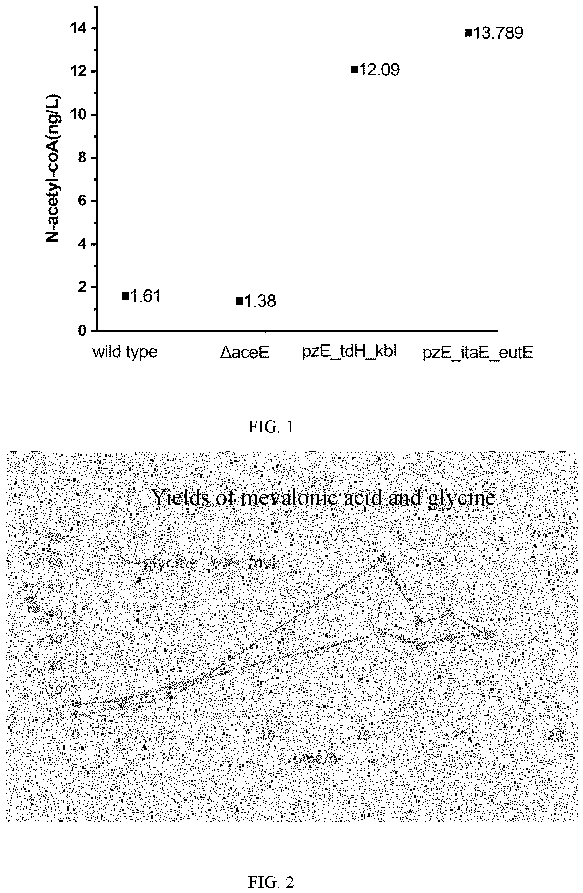

This example relates to the control test group of Example 2. The recombinant expression plasmid vectors pZE-tdH_kbI and pZE-ltaE_eutE were separately transformed into gene aceE-knocked-out *Escherichia coli* to obtain recombinant microorganisms. The two strains described above, a wild type *Escherichia coli* and the gene aceE-knocked-out *Escherichia coli* strain were separately inoculated into a 2-xyT culture medium containing ampicillin and chloramphenicol but no antibiotics. Gene aceE is a gene that is inherent in *Escherichia coli* and related to acetyl-CoA expression, and the effect of recombinant expression plasmid vectors can be more directly and clearly explained after the knockdown of gene aceE. Threonine was added at an amount of 20 g/L as a substrate, and the mixture was cultured in a 50 mL Erlenmeyer flask (medium volume 10 mL) at 30 °C and a rotation speed of 240 rpm for 3-6 h. IPTG was added to a final concentration of 0.3 mM, and the mixture was cultured for another 10 h for protein expression. The bacteria were subjected to cell wall breaking at 4 °C and centrifuged at a rotation speed of 8000 rpm for 5 min. The culture supernatant was retained, and subjected to an ice bath for further use. The detection result is shown in FIG. 1. The fermentation using the recombinant microorganism containing the recombinant expression plasmid vector pZE-tdH_kbI resulted in an acetyl-CoA concentration of 12.09 ng/L and a glycine concentration of 10.5 g/L, with a yield being 83.3%; and the fermentation using the recombinant microorganism containing the recombinant expression plasmid vector pZE-ltaE_eutE resulted in an acetyl-CoA concentration of 13.789 ng/L and a glycine concentration of 11.2 g/L, with a yield being 88.9%. While the fermentation using the wild type *Escherichia coli* only resulted in an acetyl-CoA concentration of 1.61 ng/L and a glycine concentration of 0.3 g/L; and the fermentation using the gene aceE-knocked-out *Escherichia coli* strain only resulted in an acetyl-CoA concentration of 1.38 ng/L and a glycine concentration of 0.2 g/L. It can be seen that the recombinant microorganisms described herein have great advantages in the fermentation process with threonine as a substrate.

### Example 4

This example relates to shake flask fermentation. The recombinant expression plasmid vector pZE-ltaE_eutE was transformed into gene adhE-knocked-out *Escherichia coli* to obtain a recombinant microorganism. After the gene knockout in the *Escherichia coli,* the expression activity of alcohol dehydrogenase was weakened or eliminated, which could reduce the consumption of acetyl-CoA, thereby greatly increasing the yield of products.

The recombinant microorganism was inoculated into an M9 culture medium containing ampicillin but no glucose, and threonine was added at 20 g/L (i.e., the ratio of threonine to culture medium, the same applies below). The mixture was cultured in a 50 mL Erlenmeyer flask (medium volume 10 mL, containing 0.5 g of CaCO₃ in Erlenmeyer flask) at a rotation speed of 240 rpm and 30 °C for 3 h. IPTG was added to a final concentration of 0.3 mM, the mixture was cultured and fermented for another 24 h, and then the fermentation broth was collected. It was detected that the glycine concentration was 11 g/L, with a yield being 87.3%, and the mevalonic acid concentration was 3.8 g/L, with a yield being 46.1%.

### Example 5

This example relates to fermentor fermentation. The recombinant expression plasmid vector pZE-ltaE_eutE was transformed into gene adhE-knocked-out *Escherichia coli* CMEV-1 to obtain a recombinant microorganism, and the strain was inoculated into a 50 mL LB liquid culture medium containing 100 µg/mL ampicillin for expansion culture at 37 °C and 220 rpm overnight (about 14 h). The strain was successively inoculated into an M9 culture medium containing ampicillin and loaded into a 1 L fermentor (medium volume 500 mL) for fermenting and culturing at a rotation speed of 600 rpm for 6 h. IPTG was added to a final concentration of 0.1 mM, and then threonine was added at a total amount of about 120 g/L in a continuous fed-batch addition manner, which can maintain continuous production without affecting the osmotic pressure, thereby increasing the yield. The fermentation broth was collected at different time points, and the concentrations of glycine and mevalonic acid were detected by high performance liquid chromatography. The detection result is shown in FIG. 2. It can be seen that during the fermentation, the yields of glycine and mevalonic acid peaked simultaneously at about 16 h, wherein the concentration of glycine was higher than 60 g/L, and the concentration of mevalonic acid was higher than 30 g/L, indicating an extremely excellent effect.

### Example 6

This example relates to use of acetyl-CoA integrated bacteria in the production of mevalonic acid. Strain 1 obtained by knocking out aceE in wild type *Escherichia coli* was used as a reference group.

The fragment ItaE_eutE was integrated to the position of genome aceE in wild type *Escherichia coli* by adopting a strong promoter to obtain a recombinant microorganism strain 2, avoiding the use of an inducible plasmid. The plasmid pMEV-1 was transformed into the strain with gene deletion for shake flask fermentation. The culture medium is a fermentation culture medium commonly adopted at present: glucose 4%, threonine 2%, yeast extract 0.5%, MgSO₄ 0.12%, CaCl₂ 0.01%, NH₄Cl 0.1%, NaCl 0.05%, Na₂HPO₄ 0.6%, KH₂PO₄ 0.3%, and VB1 0.0005%. The culture medium was adjusted to pH 7.0-7.2 with NaOH and hydrochloric acid, and sterilized at 115 °C for 15 min.

Culture: The strain was inoculated into an LB culture medium and cultured at 37 °C and a rotation speed of 240 rpm for 10 h. The LB culture was inoculated at an amount of 4% into a fermentation culture medium containing ampicillin, and cultured in a 125 mL Erlenmeyer flask (medium volume 10 mL, containing 0.5 g of CaCO₃) at 30 °C and a rotation speed of 240 rpm for 3 h. IPTG was then added to a final concentration of 0.3 mM, the mixture was cultured and fermented for another 36 h, and the fermentation broth was collected. It was detected that the glycine concentration was 2.8 g/L, and the mevalonic acid concentration was 5.4 g/L.

### Example 7

This example relates to a further optimization of strain 2 and shake flask fermentation. On the basis of strain 2, the genes tdh, tdcB, and yiay were deleted in sequence to obtain strain 3, strain 4, and strain 5, respectively. The plasmid pMEV-1 was transformed into strain 3, strain 4, and strain 5 separately for shake flask fermentation. The culture medium is a fermentation culture medium commonly adopted at present: glucose 4%, threonine 2%, yeast extract 0.5%, MgSO₄ 0.12%, CaCl₂ 0.01%, NH₄Cl 0.1%, NaCl 0.05%, Na₂HPO₄ 0.6%, KH₂PO₄ 0.3%, and VB1 0.0005%. The culture medium was adjusted to pH 7.0-7.2 with NaOH and hydrochloric acid, and sterilized at 115 °C for 15 min.

Culture: The strain was inoculated into an LB culture medium and cultured at 37 °C and a rotation speed of 240 rpm for 10 h. The LB culture was inoculated at an amount of 4% into a fermentation culture medium containing ampicillin, and cultured in a 125 mL Erlenmeyer flask (medium volume 10 mL, containing 0.5 g of CaCO₃) at 30 °C and a rotation speed of 240 rpm for 3 h. IPTG was then added to a final concentration of 0.3 mM, the mixture was cultured and fermented for another 36 h, and the fermentation broth was collected. It was detected that the glycine concentrations were 3.3 g/L, 3.1 g/L, and 3.1 g/L, respectively, and the mevalonic acid concentrations were 5.7 g/L, 6.2 g/L, and 6.4 g/L, respectively.

### Example 8

This example relates to a further optimization of strain 5 and shake flask fermentation. On the basis of strain 5, the ltaE_eutE gene integration was carried out in sequence at the positions of ldhA and adhE (strong promoters) to obtain strain 6 and strain 7, respectively. The plasmid pMEV-1 (selected from plasmids disclosed in the document previously published by the applicant, with the document number DOI: 10.1073/pnas.1404596) was transformed into strain 6 and strain 7 separately for shake flask fermentation. The culture medium is a fermentation culture medium commonly adopted at present: glucose 4%, threonine 2%, yeast extract 0.5%, MgSO₄ 0.12%, CaCl₂ 0.01%, NH₄Cl 0.1%, NaCl 0.05%, Na₂HPO₄ 0.6%, KH₂PO₄ 0.3%, and VB1 0.0005%. The culture medium was adjusted to pH 7.0 to 7.2 with NaOH and hydrochloric acid, and sterilized at 115 °C for 15 min.

Culture: The strain was inoculated into an LB culture medium and cultured at 37 °C and a rotation speed of 240 rpm for 10 h. The LB culture was inoculated at an amount of 4% into a fermentation culture medium containing ampicillin, and cultured in a 125 mL Erlenmeyer flask (medium volume 10 mL, containing 0.5 g of CaCO₃) at 30 °C and a rotation speed of 240 rpm for 3 h. IPTG was then added to a final concentration of 0.3 mM, the mixture was cultured and fermented for another 36 h, and the fermentation broth was collected. It was detected that the glycine concentrations were 8.1 g/L and 7.9 g/L, respectively, and the mevalonic acid concentrations were 9.6 g/L and 8.7 g/L, respectively.

### Example 9

This example relates to a further optimization of strain 7 and shake flask fermentation. The gene gcvTHP was knocked out on the basis of strain 7 to obtain strain 8. The plasmid pMEV-1 (selected from plasmids disclosed in the document previously published by the applicant, with the document number DOI: 10.1073/pnas.1404596) was transformed into strain 8 for shake flask fermentation. The culture medium is a fermentation culture medium commonly adopted at present: glucose 4%, threonine 2%, yeast extract 0.5%, MgSO₄ 0.12%, CaCl₂ 0.01%, NH₄Cl 0.1%, NaCl 0.05%, Na₂HPO₄ 0.6%, KH₂PO₄ 0.3%, and VB1 0.0005%. The culture medium was adjusted to pH 7.0-7.2 with NaOH and hydrochloric acid, and sterilized at 115 °C for 15 min.

Culture: The strain was inoculated into an LB culture medium and cultured at 37 °C and a rotation speed of 240 rpm for 10 h. The LB culture was inoculated at an amount of 4% into a fermentation culture medium containing ampicillin, and cultured in a 125 mL Erlenmeyer flask (medium volume 10 mL, containing 0.5 g of CaCO₃) at 30 °C and a rotation speed of 240 rpm for 3 h. IPTG was then added to a final concentration of 0.3 mM, the mixture was cultured and fermented for another 36 h, and the fermentation broth was collected. It was detected that the glycine concentration was 12.9 g/L, and the mevalonic acid concentration was 8.7 g/L.

### Example 10

This example relates to use of acetyl-CoA integrated bacteria in the production of N-acetyl-glucosamine (GlcNAc). The genes nagAB, nagK, and murQ were knocked out on the basis of strain 2 to obtain strain 9. The plasmid pzE-ggGy was transformed into strain 9 for shake flask fermentation. The culture medium is a fermentation culture medium commonly adopted at present: glucose 4%, threonine 2%, yeast extract 0.5%, MgSO₄ 0.12%, CaCl₂ 0.01%, NH₄Cl 0.1%, NaCl 0.05%, Na₂HPO₄ 0.6%, KH₂PO₄ 0.3%, and VB1 0.0005%. The culture medium was adjusted to pH 7.0-7.2 with NaOH and hydrochloric acid, and sterilized at 115 °C for 15 min.

Culture: The strain was inoculated into an LB culture medium and cultured at 37 °C and a rotation speed of 240 rpm for 10 h. The LB culture was inoculated at an amount of 4% into a fermentation culture medium containing ampicillin, and cultured in a 125 mL Erlenmeyer flask (medium volume 10 mL, containing 0.5 g of CaCO₃) at 30 °C and a rotation speed of 240 rpm for 3 h. IPTG was then added to a final concentration of 0.3 mM, the mixture was cultured and fermented for another 36 h, and the fermentation broth was collected. It was detected that the glycine concentration was 2.6 g/L, and the GlcNAc concentration was 5.6 g/L.

### Example 11

This example relates to a further optimization of strain 9 and shake flask fermentation. On the basis of strain 9, the ltaE_eutE gene integration was carried out in sequence at the positions of ldhA and adhE (strong promoters) to obtain strain 10 and strain 11, respectively. The plasmid pzE-ggGy was transformed into strain 10 and strain 11 separately for shake flask fermentation. The culture medium is a fermentation culture medium commonly adopted at present: glucose 4%, threonine 2%, yeast extract 0.5%, MgSO₄ 0.12%, CaCl₂ 0.01%, NH₄Cl 0.1%, NaCl 0.05%, Na₂HPO₄ 0.6%, KH₂PO₄ 0.3%, and VB1 0.0005%. The culture medium was adjusted to pH 7.0-7.2 with NaOH and hydrochloric acid, and sterilized at 115 °C for 15 min.

Culture: The strain was inoculated into an LB culture medium and cultured at 37 °C and a rotation speed of 240 rpm for 10 h. The LB culture was inoculated at an amount of 4% into a fermentation culture medium containing ampicillin, and cultured in a 125 mL Erlenmeyer flask (medium volume 10 mL, containing 0.5 g of CaCO₃) at 30 °C and a rotation speed of 240 rpm for 3 h. IPTG was then added to a final concentration of 0.3 mM, the mixture was cultured and fermented for another 36 h, and the fermentation broth was collected. It was detected that the glycine concentrations were 10 g/L and 7 g/L, respectively, and the GlcNAc concentrations were 8.1 g/L and 9.6 g/L, respectively.

The foregoing shows and describes the general principles, principal features, and advantages of the present disclosure. It should be understood by those skilled in the art that the present disclosure is not limited to the examples described above, and the examples described above and the description in the specification are merely illustration of the principles of the present disclosure. Various changes and modifications may be made without departing from the spirit and scope of the present disclosure, and such changes and modifications are within the protection scope of the present disclosure as claimed. The protection scope of the present disclosure as claimed is defined by the appended claims and equivalents thereof.

## Claims

1. A recombinant microorganism for fermentatively producing glycine by using threonine, wherein the microorganism overexpresses an endogenous or exogenous aldolase gene.

2. The recombinant microorganism as claimed in claim 1, wherein the aldolase gene comprises a threonine aldolase gene or a phenylserine aldolase gene, and the threonine aldolase gene or the phenylserine aldolase gene comprises one or more of ltaE, TdcB, glyA, Sdsl, bhcC, psald, pdxA, or vrtJ.

3. A recombinant microorganism for fermentatively producing glycine and acetyl-CoA or a derivative thereof by using threonine, wherein the recombinant microorganism at least overexpresses endogenous or exogenous aldolase gene and acetylating acetaldehyde dehydrogenase gene; and/or the recombinant microorganism at least overexpresses endogenous or exogenous threonine dehydrogenase gene and 2-amino-3-ketobutyrate CoA ligase gene; optionally, the expression activity of an alcohol dehydrogenase of the microorganism is weakened or eliminated, and encoding genes for the alcohol dehydrogenase are one or more of yqhD, qbdA, adhE, or mdH;
optionally, the expression activity of a pyruvate dehydrogenase or a lactate dehydrogenase of the microorganism is weakened or eliminated, and the pyruvate dehydrogenase or the lactate dehydrogenase is aceE or ldhA;
preferably, the aldolase gene comprises one or more of ltaE, TdcB, glyA, Sdsl, bhcC, psald, pdxA, or vrtJ; the acetylating acetaldehyde dehydrogenase gene comprises one or more of eutE, bphJ, TTHB247, mhpF, ADH2, or hsaG; the threonine dehydrogenase gene comprises one or more of tdh, ydfG, pdxA, asd, AKHSDH2, or trmG; and the 2-amino-3-ketobutyrate CoAligase gene comprises one or more of kbI, GCAT, Gcat, or TTHA1582;
preferably, the aldolase gene, the acetylating acetaldehyde dehydrogenase gene, the threonine dehydrogenase gene, or the 2-amino-3-ketobutyrate CoA ligase is introduced into a genomic gene;
preferably, the aldolase gene, the acetylating acetaldehyde dehydrogenase gene, the threonine dehydrogenase gene, or the 2-amino-3-ketobutyrate CoA ligase is initiated by a strong promoter;
preferably, the aldolase gene, the acetylating acetaldehyde dehydrogenase gene, the threonine dehydrogenase gene, or the 2-amino-3-ketobutyrate CoA ligase is integrated to positions of aceE, ldhA, or/and adhE;
preferably, Tdh, tdcB, and Yiay genes are deleted in the microorganism;
preferably, gcvTHP gene is deleted in the microorganism.

4. The recombinant microorganism as claimed in claim 3, wherein
the microorganism is used for synthesizing mevalonic acid and expresses three genes of AtoB, MvaS and MvaE by plasmid expression or genome integration;
or
the microorganism is used for synthesizing N-acetyl-glucosamine (Glcnac); nagAB, nagK, and murQ are deleted in the microorganism and the microorganism comprises the following genes: one or any combination of glutamine fructose-6 phosphate transaminase glmS, phosphatase gene yqaB, glutamine synthetase gene glnA, or acetylglucosamine synthetase GNA1.

5. The microorganism as claimed in any one of claims 1-4, wherein the recombinant microorganism is constructed by a genetic engineering method including plasmid expression or genomic integration.

6. The microorganism as claimed in claim 5, wherein the recombinant microorganism is constructed by the plasmid expression method, and the construction method is as follows: the gene is amplified by PCR, the obtained gene is ligated to a plasmid vector, and the plasmid vector is then transformed into competent cells; and after sequencing, a recombinant expression plasmid vector is obtained and then transformed into a microorganism to obtain the recombinant microorganism.

7. The microorganism as claimed in claim 6, wherein the plasmid is one or two of pZAlac and pZElac, and the competent cells are *E.coli* dh5a competent cells.

8. The microorganism as claimed in claim 7, wherein the recombinant expression plasmid vector is pZE-ltaE, and the construction method for the plasmid pZE-ltaE is as follows: an ltaE gene is obtained by PCR amplification using a genome of *Pseudomonas putida* as a template; the ltaE gene is ligated to a pZElac vector comprising an IPTG inducible promoter, and the vector is transformed into *E.coli* dh5a competent cells; and the plasmid pZE-ltaE is obtained after sequencing.

9. The microorganism as claimed in claim 7, wherein the recombinant expression plasmid vector is pZE-ltaE_eutE, and the construction method for the pZE-ltaE_eutE is as follows: an eutE gene is obtained by PCR amplification using a genome of *Escherichia coli* MG1655 as a template, and an ltaE gene is obtained by PCR amplification using a genome of *Pseudomonas putida* as a template; the eutE and ltaE genes are ligated to a pZElac vector comprising an IPTG inducible promoter, and the vector is transformed into *E.coli* dh5a competent cells; and the plasmid pZE-ltaE_eutE is obtained after sequencing;
and/or
the recombinant expression plasmid vector is pZE-tdH kbI, and the construction method for the pZE-tdH_kbI is as follows: tdH and _kbI genes are obtained by PCR amplification using a genome of *Escherichia coli* MG1655 as a template; the tdH and _kbI genes are ligated to a pZElac vector comprising an IPTG inducible promoter, and the vector is transformed into *E.coli* dh5a competent cells; and the plasmid pZE-tdH_kbI is obtained after sequencing.

10. The recombinant microorganism as claimed in any one of the preceding claims, wherein the microorganism is selected from one or more of *Escherichia coli, Bacillus, Corynebacterium, yeast,* or *Streptomyces.*

11. The microorganism as claimed in claim 10, wherein the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

12. Use of the recombinant microorganism as claimed in any one of claims 1-2 or 5-8 for fermentatively producing glycine by using threonine.

13. A method for preparing glycine by using threonine, wherein threonine is used as a substrate and the recombinant microorganism as claimed in any one of claims 1-2 or 5-11 is added for fermentation; and during the fermentation, the threonine is decomposed into glycine and acetaldehyde.

14. The method for preparing glycine by using threonine as claimed in claim 13, wherein during the fermentation, a strain of the recombinant microorganism is inoculated into a 2-xyT culture medium containing ampicillin and chloramphenicol for culturing for 3-6 h, and then IPTG is added to a final concentration of 0.2-0.4 mM; the mixture is cultured for another 15-30 h and then centrifuged to obtain a bacterial solution; the bacterial solution is added to a Tris-HCI buffer, and threonine is added at an amount of 15-30 g/L as a substrate; the resulting mixture is fermented to obtain a fermentation broth containing glycine and acetaldehyde.

15. Use of the recombinant microorganism as claimed in any one of claims 3-7 or 9-11 for fermentatively producing glycine and acetyl-CoA or a derivative thereof by using threonine.

16. A method for preparing glycine and acetyl-CoA by using threonine, wherein threonine is used as a substrate and the recombinant microorganism as claimed in any one of claims 3-7 or 9-11 is added for fermentation; during the fermentation, the threonine is decomposed into glycine and acetaldehyde, and the acetaldehyde is directly converted into acetyl-CoA or the acetaldehyde is converted into acetic acid first and then into acetyl-CoA; or the threonine is dehydrogenated to obtain 2-amino-3-ketobutyric acid, and the 2-amino-3-ketobutyric acid is subjected to the action of a 2-amino-3-ketobutyrate CoA ligase to obtain acetyl-CoA.

17. The method for preparing glycine and acetyl-CoA by using threonine as claimed in claim 16, wherein during the fermentation, the recombinant microorganism is inoculated into a 2-xyT culture medium containing ampicillin and chloramphenicol but no antibiotics, and threonine is added at an amount of 15-25 g/L as a substrate; the mixture is cultured at 30-35 °C for 3-6 h, and then IPTG is added to a final concentration of 0.2-0.4 mM; the resulting mixture is cultured for another 10-12 h and then centrifuged after the culture is finished, and a supernatant is retained, thus obtaining glycine and acetyl-CoA.

18. A method for preparing glycine and an acetyl-CoA derivative by using threonine, wherein glycine and acetyl-CoA are prepared by the method as claimed in claim 16, and the acetyl-CoA is converted into an acetyl-CoA derivative.

19. The method for preparing glycine and an acetyl-CoA derivative by using threonine as claimed in claim 18, wherein the acetyl-CoA derivative comprises one or more of mevalonic acid, sialic acid, N-acetylglucosamine, 3-hydroxybutyric acid, or fatty acid;
preferably, the derivative is mevalonic acid or N-acetylglucosamine, and the microorganism is the microorganism as claimed in claim 4.

20. The method for preparing glycine and an acetyl-CoA derivative by using threonine as claimed in claim 19, wherein the acetyl-CoA derivative is mevalonic acid; during the fermentation, the recombinant microorganism is inoculated into a shake flask containing an M9 culture medium containing ampicillin but no glucose, and threonine is added at an amount of 15-25 g/L as a substrate; the mixture is cultured at 30-35 °C and a rotation speed of 200-300 rpm for 3-5 h, and then IPTG is added to a final concentration of 0.2-0.4 mM; the resulting mixture was cultured and fermented for another 20-30 h, and a fermentation broth is collected.

21. The method for preparing glycine and an acetyl-CoA derivative by using threonine as claimed in claim 19, wherein the acetyl-CoA derivative is mevalonic acid; during the fermentation, the recombinant microorganism is subjected to an expansion culture and then inoculated into a fermentor containing an M9 culture medium containing ampicillin but no glucose; after 6-8 h of fermentation and culture, IPTG is added to a final concentration of 0.1-0.2 mM, and threonine is added at an amount of 100-150 g/L as a substrate; the resulting mixture is fermented and cultured at 30-35 °C for another 15-20 h, and a fermentation broth is collected.

22. A recombinant DNA or biomaterial for fermentatively producing glycine and acetyl-CoA or a derivative thereof, wherein the recombinant DNA or biomaterial at least comprises an aldolase gene and an acetylating acetaldehyde dehydrogenase gene, and/or the recombinant DNA or biomaterial at least comprises a threonine dehydrogenase gene and a 2-amino-3-ketobutyrate CoA ligase gene;
preferably, the aldolase gene comprises one or more of ltaE, TdcB, glyA, Sdsl, bhcC, psald, pdxA, or vrtJ; the acetylating acetaldehyde dehydrogenase gene comprises one or more of eutE, bphJ, TTHB247, mhpF, ADH2, or hsaG; the threonine dehydrogenase gene comprises one or more of tdh, ydfG, pdxA, asd, AKHSDH2, or trmG; and the 2-amino-3-ketobutyrate CoAligase gene comprises one or more of kbI, GCAT, Gcat, or TTHA1582;
the biomaterial is an expression cassette, a transposon, a plasmid vector, a phage vector, or a virus vector.
